# EUROPÄISCHE PATENTSCHRIFT

(11) **EP 1 169 461 B1**
(45) Veröffentlichungstag und Bekanntmachung des Hinweises auf die Patenterteilung: **04.10.2006**
(21) Anmeldenummer: 00926751.9
(22) Anmeldetag: 23.03.2000
(51) Int. Cl.: C12N 15/57, C12N 9/48, C12N 1/19, C07K 14/62

(54) **Verwendung von pankreatischer Procarboxypeptidase B zur Herstellung von Insulin**
Use of pancreatic procarboxypeptidase B for the production of insulin
Utilisation de procarboxypeptidase B pancréatique pour la production d'insuline

(30) Priorität: 09.04.1999 DE 19915938
(43) Veröffentlichungstag der Anmeldung: 09.01.2002
(73) Patentinhaber: Sanofi-Aventis Deutschland GmbH, 65929 Frankfurt am Main (DE)
(72) Erfinder: HABERMANN, Paul, D-65817 Eppstein (DE)
(86) Internationale Anmeldenummer: PCT/EP2000/002571
(87) Internationale Veröffentlichungsnummer: WO 2000/061727

(56) Entgegenhaltungen:
- WO-A-95/14096
- WO-A-96/02637
- WO-A-96/23064
- HOCULI E ET AL: "GENETIC APPROACH TO FACILITATE PURIFICATION OF RECOMBINANT PROTEINS WITH A NOVEL METAL CHELATE ADSORBENT" BIO/TECHNOLOGY,US,NATURE PUBLISHING CO. NEW YORK, November 1988 (1988-11), Seiten 1321-1325, XP002916185 ISSN: 0733-222X
- REVERTER DAVID ET AL: "Overexpression of human procarboxypeptidase A2 in Pichia pastoris and detailed characterization of its activation pathway." JOURNAL OF BIOLOGICAL CHEMISTRY, Bd. 273, Nr. 6, 6. Februar 1998 (1998-02-06), Seiten 3535-3541, XP002148710 ISSN: 0021-9258

## Beschreibung

Carboxypeptidasen sind eine Gruppe von Proteine bzw. Peptide spaltenden, zinkhaltigen Enzymen (Proteasen), durch die einzelne Aminosäuren schrittweise vom Carboxyterminus der abzubauenden Proteine bzw. Peptide hydrolytisch abgespalten werden. Carboxypeptidasen zählen daher zu den Exopeptidasen. Tierische Carboxypeptidasen werden in der Bauchspeicheldrüse als Vorstufen (Procarboxypeptidasen) gebildet und im Darm durch Trypsin in die aktiven Formen umgewandelt, wo sie für die Verdauung von Peptiden, den primären Spaltprodukten der Proteine, von großer Bedeutung sind. Nach der Substratspezifität unterscheidet man zwischen verschiedenen Carboxypeptidasen.

Carboxypeptidase B setzt z. B. ausschließlich basische Aminosäuren, wie Arginin, Lysin und Omithin, frei. Carboxypeptidasen sind Hilfsmittel bei der Sequenzermittlung von Peptiden und Proteinen, da mit ihrer Hilfe die an den Carboxyenden stehenden Aminosäuren identifiziert werden können. Außerdem lassen sich durch Einsatz von Carboxypeptidase B Proteine maßschneidern.

Ein technisches Beispiel für den Einsatz von Carboxypeptidase B ist die Herstellung des wichtigen pharmazeutischen Produkts Insulin. Die Herstellung dieses Peptidhormons ist u.a. in der Europäischen Patentanmeldung EP-A 0 489 780 beschrieben. Dabei kommt Carboxypeptidase B in einem wichtigen Schritt bei der Umwandlung von mit Trypsin geöffneten Proinsulinstrukturen zu Insulinen zum Einsatz.

Die Carboxypeptidase B, die in solchen technischen Verfahren zum Einsatz kommt, stammt i.d.R. aus Carboxypeptidase B Präparationen, die kommerziell erhältlich sind. Diese werden bevorzugt aus Schweinepankreata gewonnen ( Folk, J.E., Meth. Enzym.: 19, 504, 1970).

Enzyme tierischen Ursprungs haben jedoch den Nachteil, daß sie mit dem Risiko der Kontamination mit tierischen Viren behaftet sein können. Der Nachweis der Virenfreiheit ist aufwendig und geht als ein wesentlicher Faktor in die Kalkulation der Herstellkosten ein. Wenn das Enzym in großtechnischen Verfahren, wie der industriellen Insulinherstellung Verwendung findet, liegt ein weiterer Nachteil in hohen Logistikkosten zur Beschaffung und Aufbewahrung gefrorener Pankreata.

Als Alternative zur Herstellung von Carboxypeptidase B über die Extraktion von Pankreasgewebe bietet sich die biotechnologische Gewinnung an. Dabei gibt es mehrere bekannte Herstellwege, wie die direkte intrazelluläre Expression oder die Expression über ein Fusionsprotein in Bakterien, z.B. in Form eines β-Galaktosidase-Carboxypeptidase B Fusionsproteins (J. Biol. Chem., 267:2575-2581, 1992) oder entsprechende Expression in Hefen. Eine weitere Alternative dazu ist die Expression eines Carboxypeptidase B - Vorläufers, der Procarboxypeptidase B, welcher aus der Aminosäuresequenz der Carboxypeptidase B zuzüglich einer Signalsequenz besteht, die zur Sekretion des Expressionsprodukts führt. Geeignete Expressionssysteme wurden für *Bacillus subtilis, Streptomyces, E.coli* und die Hefen *Saccharomyces, Candida, Hansenula polymorphus* und *Pichia pastoris* beschrieben und sind teilweise kommerziell erhältlich.

Voraussetzung für die Anwendung von Expressionssystemen zur Herstellung von Carboxypeptidase B ist es, daß der jeweils gewählte Wirtstamm durch die Gegenwart aktiver Carboxypeptidase B nicht in seinem Wachstum gehemmt wird, so daß eine Expression überhaupt erst möglich wird. Wirtsstämme, die diese Eigenschaft aufweisen, sind meist schwierig zu fermentieren, so daß sich eine schlechtere Ausbeute über Raum und Zeit einstellt.

Ein Problem bei der intrazellulären Expression von Carboxypeptidase B (Direktexpression oder als Fusionsprotein) liegt darin, daß das Protein zunächst nicht in der richtigen räumlichen Struktur vorliegt und somit inaktiv ist. Es muß dann während der Reinigung und Prozessierung *in vitro* gefaltet werden. Dabei kann es zu Fehlfaltungen kommen, die sich auf die Aktivität und Spezifität des Enzymes negativ auswirken und den Einsatz in der Arzneimittelherstellung erschweren.

Auch die Herstellung von Carboxypeptidase B durch Sekretion der reifen, aktiven Form des Enzyms zeigt Nachteile. Während der Fermentation wird Carboxypeptidase B durch die Umsetzung mit peptidartigen Bruchstücken zellulärer Bestandteile oder Bestandteile des Nährmediums, die als Substrat erkannt werden, laufend inaktiviert, so daß Ausbeuteverluste auftreten.

In der internationalen Patentanmeldung mit der Veröffentlichungsnummer WO95/14096 sind DNA-Sequenzen offenbart, die für Carboxypeptidase vom Schwein kodieren. Von Hochuli et al. (Bio/Technology, Nature Publishing Co., New York, November 1988, pages 1321-1325) wurde ein neuer genetischer Ansatz beschrieben, um die Reinigung rekombinanter Proteine mittels Metallchelat-Absorbentien vorzunehmen.

Ein Ausweg aus diesem Dilemma ist die Expression und Sekretion einer inaktiven Carboxypeptidase B - Form in räumlich korrekter Form. Durch Umsetzung mit einem Enzym läßt sich aus diesem Vorläufer *in vitro* die aktive Carboxypeptidase B herstellen. "Aktive Carboxypeptidase B" bedeutet hierbei eine Carboxypeptidase B, die sich in der Natur findet, z.B. Carboxypeptidase B des Menschen oder natürlich vorkommende lsoformen davon. "Aktive Carboxypeptidase B" kann ebenfalls ein Mutein der natürlich vorkommenden Carboxypeptidase B bedeuten, bei dem Deletionen, Additionen oder Substitutionen der Aminosäuresequenz vorkommen, die enzymatische Aktivität des Muteins aber qualitativ der enzymatischen Aktivität von natürlich vorkommender Carboxypeptidase B entspricht. Der genannte Vorläufer kann die natürliche Procarboxypeptidase B oder ein Derivat hiervon sein, z.B. eine Isoform oder ein Mutein oder Carboxypeptidase B, die durch Hinzufügung einer Peptidsequenz inaktiviert wurde. Die Art der hinzugefügten Proteinsequenz wird im weiteren näher beschrieben. Bevorzugt ist Procarboxypeptidase B oder ein Derivat hiervon, da sich aus diesem Protein überraschend gut steuerbar die aktive Carboxypeptidase B herstellenläßt. Zudem können Procarboxypeptidase B und die genannten Derivate davon über längere Zeiträume gelagert werden, während man bei der Lagerung von Carboxypeptidase B einen Verlust an Aktivität beobachtet.

Die genannten Derivate von Procarboxypeptidase B enthalten die Aminosäuresequenz von Carboxypeptidase B zuzüglich eines N-terminal angefügten Peptides mit der Aminosäuresequenz eines Signalpeptides zur Ausschleusung des Derivates aus dem zur Expression benutzten Wirtsorganismus, ggf. einer beliebigen, bis zu 100 Aminosäuren langen Aminosäuresequenz und einer Endopeptidaseerkennungssequenz, welche die enzymatische Abspaltung des zuzüglich N-terminal angefügten Peptides von dem Carboxypeptidase B-Anteil des Derivats ermöglicht. Beispiele für solche Endopeptidaseerkennungssequenzen sind die entsprechenden bekannten Erkennungssequenzen von Trypsin, Faktor Xa, Elastase, Chymotrypsin und Collagenase.

Überraschend wurde gefunden, daß sich Procarboxypeptidase B mit Proinsulin und Trypsin in einer Eintopfreaktion umsetzen läßt, so daß die frisch gebildete aktive Carboxypeptidase B sofort die entstehenden carboxyterminalen Arginine der Insulin B-Kette erkennt und hydrolysiert. Die während des Prozesses entstehende Carboxypeptidase B ist aktiver als solche Carboxypeptidase B, die vorher gelagert und dann zur Umsetzung mit Proinsulin hinzugegeben wurde. Das im Reaktionsgemisch vorhandene Trypsin dient nicht nur zur Aktivierung von Procarboxypeptidase B, sondern schneidet auch Proinsulin spezifisch und trägt somit zur Freisetzung reifen Insulins bei.

Die Aktivierungskinetik durch Trypsin wird überraschend durch die Addition einer Tetra - His -Sequenz am N -Terminus der Procarboxypeptidase B oder die genannten Derivate nicht beinträchtigt. Der Vorteil einer solchen Addition liegt darin, daß das Protein so mittels Nickel - Chelatkomplexierung leicht affinitätschromatographisch gereinigt werden kann. Die Verwendung solcher modifizierter Procarboxypeptidase B ist ebenfalls Gegenstand der Erfindung.

Beispielhaft für die Erfindung wird die cDNA - Sequenz humaner pankreatischer Procarboxypeptidase B exprimiert. Es ist jedoch klar, daß aufgrund der großen Sequenzhomologie, sowohl auf DNA - als auch auf Proteinebene, zwischen dem humanen Enzym und den entsprechenden Enzymen, z.B.aus Rind, Ratte, Schwein oder anderen Spezies,die Pankreasgewebe bilden, statt der humanen DNA - Sequenz die DNA - Sequenz solcher Spezies ebenfalls verwendet werden könnte. Auch kann die entsprechende DNA kodierend für Muteine der Carboxypeptidase B verwendet werden. Ebenso können DNA - Sequenzen, die für natürliche oder künstlich erzeugte Isoformen der Carboxypeptidase B oder der Procarboxypeptidase B kodieren, verwendet werden. Außerdem können natürlich alle diejenigen nicht natürlich vorkommenden DNA-Sequenzen verwendet werden, die auf Grund der Degeneriertheit des genetischen Codes die vorgenannten DNA-Sequenzen kodierend eine Carboxypeptidase B oder Procarboxypeptidase B oder jeweils ein Mutein davon ersetzen können.

Ebenfalls exemplarisch wird das über die Firma Invitrogen kommerziell erhältliche *Pichia pastoris* Expressionssystem zur Sekretion heterologer Proteine zur Herstellung der humanen Procarboxypeptidase B verwendet. Es ist dem Fachmann klar, daß auch bakterielle Systeme, z. B. *E. coli* Sekretormutanten, wie in der Europäischen Patentanmeldung EP-A- 0 448 093 beschrieben wurden, verwendet werden können, wenn man die dort beschriebene Hirudinsequenz durch die Sequenz der Procarboxypeptidase ersetzt. Eine weitere Alternative wäre die Expression der humanen Procarboxypeptidase B in Streptomyceten, wie z.B. in der Europäischen Patentanmeldung EP-A 0 504 798 für den Fall der Expression von Glutarylamidase beschrieben.

Ebenfalls ist klar, daß bei der großtechnischen Verwendung des Verfahrens zur Isolation größerer Mengen Enzym andere proteintechnische Reinigungsschritte als in den Beispielen beschrieben, wie sie Stand der Technik sind, Verwendung finden können.

Demgemäß erfordert die Erfindung ein Verfahren zur Herstellung pankreatischer Carboxypeptidase B oder einer Isoform oder eines Muteins der Carboxypeptidase B, wobei
(a) eine natürliche oder unnatürliche Vorläuferform von Carboxypeptidase B oder einer Isoform oder eines Muteins der Carboxypeptidase B in einem Mikroorganismus sekretorisch exprimiert,
(b) die sekretorisch exprimierte Vorläuferform gereinigt und
(c) die gereinigte Vorläuferform durch eine enzymatische Behandlung in die aktive Carboxypeptidase B oder eine Isoform oder ein Mutein der Carboxypeptidase B überführt wird;
insbesondere ein solches Verfahren, bei dem die pankreatische Carboxypeptidase B oder eine lsoform davon aus dem Menschen stammt, bevorzugt ein solches Verfahren bei dem die natürliche Vorläuferform der Procarboxypeptidase B oder einer Isoform davon entspricht.

Im weiteren wird bei der Bezugnahme auf das beschriebene Verfahren i.d.R. der Ausdruck "ein solches Verfahren" verwendet, sofern nicht ausdrücklich auf ein anderes Verfahren hingewiesen wird. "Natürliche" und "unnatürliche" Vorläuferformen von Carboxypeptidase B sind solche Moleküle, die in der Natur vorkommen ("natürliche Vorläuferformen") oder von solchen natürlichen Vorläuferformen durch Substitution, Addition oder Deletion von Aminosäuren hervorgegangen sind ("unnatürliche Vorläuferform"), wobei jedoch die unnatürlichen Vorläuferformen durch eine enzymatische Behandlung in aktive pankreatische Carboxypeptidase B oder Isoformen oder Muteine davon übergeführt werden können.

Besonders bevorzugt ist ein solches Verfahren, bei dem die unnatürliche Vorläuferform folgende Struktur hat:

S-(As)ₓ-E-CB, (I)

wobei
- S: ein Signalpeptid ist, welches die Sekretion des bei der Expression entstehenden Fusionsproteins aus dem jeweiligen Mikroorganismus bewirkt;
- As: eine beliebige genetisch kodierbare Aminosäure ist;
- E: ein peptidischer Linker bestehend aus einer Endopeptidaseerkennungssequenz ist;
- CB: die Aminosäuresequenz der Carboxypeptidase B oder einer Isoform oder eines Muteins der Carboxypeptidase B ist; und
- x: eine ganze Zahl von 0 - 100 ist.

Desweiteren bevorzugt ist ein solches Verfahren, bei dem an die natürliche oder unnatürliche Vorläuferform eine Peptidsequenz angefügt ist, welche die affinitätschromatographische Reinigung der Vorläuferform ermöglicht, besonders bevorzugt ist ein solches Verfahren, bei dem die für die affinitätschromatographische Reinigung angefügte Sequenz 1 bis 6, vorzugsweise 4 Histidinreste darstellen.

Ebenfalls bevorzugt ist ein solches Verfahren, wobei als Mikroorganismus zur Expression die Hefe *Pichia pastoris* verwendet wird.

Zusätzlich bevorzugt ist ein solches Verfahren, bei dem zur enzymatischen Behandlung die Enzyme Trypsin, Elastase, Faktor Xa, Chymotrypsin oder Collagenase, vorzugsweise Trypsin, verwendet werden.

Gegenstand der Erfindung ist ein solches Verfahren, bei dem Schritt (c) unter geeigneten Reaktionsbedingungen in Gegenwart einer Insulinvorläuferform enthaltend die B, C und A-Ketten von Insulin oder eines Insulinderivats abläuft und hierbei reifes Insulin oder ein reifes Insulinderivat entsteht, welches aus dem Reaktionsgemisch isoliert wird.

Ein weiterer Gegenstand der Erfindung ist die Verwendung von Procarboxypeptidase B und Carboxypeptidase B in einem solchen Verfahren.

Die vorliegenden Beispiele sollen veranschaulichen, wie man Procarboxypeptidase B und (His)4-Procarboxypeptidase B exprimieren und reinigen könnte. Darüberhinaus wird erläutert, wie Procarboxypeptidase B mit Trypsin aktiviert werden könnte, und zwar sowohl in isolierter Form als auch beim kombinierten Einsatz von Trypsin und Procarboxypeptidase B zur Herstellung von Insulin.

### Beispiel 1

Das Beispiel beschreibt die Herstellung eines rekombinanten *P. pastoris* Stammes zur Sekretion von humaner Procarboxypeptidase B. Als Ausgangsmaterial dient eine cDNA Präparation wie sie gemäß bekannter Methoden aus RNA, die aus humanem Pankreasgewebe isoliert wurde, hergestellt wird. Solche cDNA Präparationen sind kommerziell z.B. von der Firma Clontech (Katalog Nr. 7410-1) erhältlich. Zur Amplifikation der gewünschten cDNA Zielsequenz werden zwei Primer synthetisiert. Die Sequenzen dazu werden der Genbank Datenbank entnommen. Dort ist die cDNA Sequenz für humane pankreatische Carboxpeptidase B unter der 'Accession Nummer' M81057 zugänglich. Die vorwärts gerichtete Primersequenz P-CPBf22 entspricht dem Bereich 22bp - 32 bp und die rückwärts gerichtete Primersequenz P-CPBrev1271 der Region 1271 bp - 1250 bp. Zur Amplifikation wird eine Standard Polymerase Ketten Reaktion (PCR) durchgeführt. Die Reaktionsprodukte der PCR werden gelelektrophoretisch aufgetrennt und die entstandene DNA- Bande der erwarteten Länge von ca. 1250 bp eluiert und in einer Ligationsreaktion mit dem PCR® Vector des Original TA Cloning ®-Kits der Firma Invitrogen umgesetzt. Kompetente Zellen des als Bestandteil des Kits mitgelieferten Stammes *E.coli* INVα F' werden mit dem Ligationsgemisch transformiert und auf NA - Platten, die 25 mg/Liter Ampicillin enthalten, ausplattiert und bei 37 ° C inkubiert. Von den gewachsenen Kolonien werden am nächsten Morgen Zellen entnommen in 20µl steriles Wasser resuspendiert und für 10 Minuten bei 94 ° C inkubiert. Der Suspension wird anschließend PCR - Reaktionspuffer, der je 0,2µg der beiden beschrieben Primer P-CPBf22 und P-CPBrev1271 enthält, so zugegeben, daß eine Standard PCR in einem Reaktionsvolumen von 100 µl durchgeführt werden kann. Die Reaktionsprodukte werden anschließend geleelektrophoretisch analysiert. Transformanten, die DNA enthalten, die zu einem ca. 1250 bp Fragment amplifiziert werden können, werden als richtig erkannt. Von einem der so definierten Klone wird Plasmid DNA isoliert und die insertierte cDNA - Sequenz mittels Sequenzanalyse vollständig charakterisiert. Es zeigt sich, daß die ermittelte Sequenz vollständig identisch zu der von Aloy *et al.* (Biol. Chem., 379:149 - 155, 1998 ) publizierten Sequenz ist. Entsprechend der Publikation dienen die Codone, welche die Aminosäuren -95 bis 307 der Procarboxypeptidase kodieren, zur Expression. Als Expressionsvektor dient das Plasmid pPIC9, das von Cregg, J.M. *et al.* (Bio/Technologie, 11:905-910, 1993) und Scorer, C.A. *et al.* (Bio/Technologie, 12:181 -184, 1994) beschrieben wurde. Das Plasmid pPIC9 wird dazu mit den Restriktionsenzymen XhoI und EcoRI geöffnet. Zur Insertion der cDNA Sequenz in den Vektor werden zwei Primer synthetisiert. Der vorwärtsgerichtete Primer PPICCPBf hat die Sequenz 
XhoI Procarboxypeptidase →

5´ TTTTTTCTCGAGAAAAGACATCATGGTGGTGAGCAC 3´ (SEQ ID NO.: 1)
 und der rückwärtsgerichtete Primer PPICCPBrev die Sequenz

EcoRI Procarboxypeptidase
5´ TTTTTTGAATTCCTTACTACTAGTACAGGTGTTCCAGGAC 3´
(SEQ ID NO.: 2)

In einer Standard PCR Reaktion wird die cDNA mit den beiden Primern amplifiziert und das entstandene Fragment nach Reinigung mit den Enzymen *Xho*I und *Eco*RI verdaut. Das so maßgeschneiderte Fragment wird aus dem Reaktionsgemisch ausgefällt, in Wasser aufgenommen und mit dem geöffneten Vektorfragment in einer Ligasereaktion umgesetzt. Kompetente INVαF' Zellen werden mit dem Ligationsgemisch transformiert und auf Selektionsplatten ausplattiert. Kolonien, die das gewünschte Expressionsplasmid enthalten, werden wie beschrieben mittels PCR - Technologie identifiziert. Von einem als richtig erkannten Klon wird Plasmid DNA gewonnen. Diese DNA wird wie von Cregg J.M. *et al.* beschrieben in den für Histidin auxotrophen *P. pastoris* Stamm GS115 (Scorer C.A. *et al.* Biotechnology,12:181-184,1994) eingeführt. Kolonien, die nach Transformation prototroph für Histidin geworden sind, werden auf Expression des Procarboxypeptidaseproteins untersucht. Dazu werden 50 Klone wie von Clare, J. J. *et al.* (Gene, 105:205 - 212, 1991) beschrieben exprimiert. Am Ende der Expression wird 1 ml Kulturmedium entnommen, die Zellen abzentrifugiert und der klare Überstand gefriergetrocknet. Aliquots des Überstandes werden mittels SDS - Polyacrylamidgelelektrophrese analysiert. Nach Coomassie Blaufärbung sieht man, daß bei Proben einiger der Überstände im Bereich von 45000 dt Molekulargewicht eine deutliche Bande sichtbar ist, die in Proben von Überständen nicht induzierter Kulturen nicht beobachtet wird. Diese Bande wird in der Western Blot Analyse von dem Anti-Schweinecarboxypeptidase B Antikörper der Firma Chemicon (Bestell Nr. AB1801) deutlich erkannt. Von dem Klon, der in diesem Experiment die beste Ausbeute liefert, wird in einem 2l Kreuzkolben eine 100 ml Kultur kultiviert, die Procarboxypeptidase B exprimiert und gemäß Beispiel-2 isoliert.

### Beispiel 2

Das Beispiel beschreibt die Reinigung von humaner Procarboxypeptidase B aus Überständen von Kulturbrühen gemäß Beispiel 1. Zunächst werden die Zellen abzentrifugiert. Der klare Überstand wird anschließend mit Ammoniumsulfat versetzt bis eine ungefähr 55 %-ige Sättigung erreicht ist. Das ausgefällte Protein wird abzentrifugiert und das Pellet in 5 ml einer 50 mM Tris - HCl (pH 7,5) Lösung enthaltend 1 mM EDTA, gelöst. Die Proteinsuspension wird anschließend über DEAE - Cellulose - Chromatographie aufgetrennt. Das Elutionschromatogramm wird über einen 0 bis 0,5 M NaCl Gradienten erstellt. Die Fraktionen, die das gewünschte Protein enthalten, werden über Western - Blot Analyse identifiziert. Die Fraktionen werden vereinigt und über Ultrafiltration konzentriert. In dem Retentat wird mittels Proteinbestimmung nach Bradford die Konzentration an Protein bestimmt. Die so angereicherte Procarboxypeptidase wird anschließend zur Lagerung gefriergetrocknet oder direkt gemäß Beispiel 4 durch Trypsinbehandlung aktiviert. Die Coomassie Blaufärbung des gelelektrophoretisch aufgetrennten Materials zeigt, daß ungefähr 60 % des Materials in einer Proteinbande von ca. 45000 dt Molekulargewicht gefunden werden. Die Bande korrespondiert mit dem im Western Blot identifizierten Bereich.

### Beispiel 3

Das Beispiel beschreibt die Herstellung des Expressionsvektors zur Synthese von (His ) ₄ - Procarboxypeptidase B. Die Konstruktion erfolgt entsprechend dem im Beispiel 1 beschriebenen Weg. Verwendet wird der Primer PPICCPBrev (siehe Beispiel 1). Der vorwärtsgerichtete Primer wird so modifiziert, daß er vier zusätzliche Codone für Histidin enthält. Die Sequenz des Primers PCPBHisf lautet entsprechend :

XhoI Procarboxypeptidase →
5´ TTTTTTCTCGAGAAAAGACACCATCACCACCATCATGGTGGTGAGCAC 3´
( His )4
(SEQ ID NO.: 3)

Der so konstruierte *P.pastoris* Stamm wird zur Expression benutzt und das His - Procaboxypeptidase B - Protein nach Fällung mit Ammoniumsulfat in Lösung über einen Nickelaffinitätschromatographieschritt direkt gereinigt. Als Chromatographieträgermaterial wird "ProBond^{™} NICKEL Chelating Resin" (Invitrogen Katalog Nr. R801-01) entsprechend der Angaben des Herstellers verwendet. Die gelelektrophoretische Analyse ergibt nach Coomassie Blaufärbung praktisch nur eine sichtbare Bande, die ein Molekulargewicht von ca. 45000 dt aufweist. Diese Bande wird im Western Blot Experiment von dem eingesetzten Antikörper erkannt. Das so gereinigte Material wird ultrafiltriert und nach Proteinbestimmung nach der Methode von Bradford entweder zur Lagerung gefriergetrocknet oder direkt gemäß Beispiel 4 aktiviert.

### Beispiel 4

Das Beispiel beschreibt die Aktivierung von Procarboxypeptidase B durch Umsetzung mit Trypsin. Dazu werden 22mg gefriergetrocknetes Material aus Beispiel 2 in 14 ml einer 0,1 molaren Tris -HCl - Lösung (pH 7,8) gelöst, auf 26 °C erwärmt und mit 15 µl einer Trypsinlösung ( 0,1 U/ml ) versetzt und 3 Stunden unter Rühren inkubiert. Anschließend wird die Lösung mit einem Trypsininhibitor aus der Sojabohne versetzt und das Trypsin, der Inhibitor, die von der Carboxypeptidase B abgespaltenen Propeptidfragmente und andere Bestandteile durch Mikrofiltration über eine Centriprep ® - Filtereinheit (Firma Amicon) mit einer Molekulargewichtsausschlußgrenze von 30000 dt von der Carboxypeptidase B abgetrennt. Die aktive Carboxypeptidase B wird in einem 5mM Tris - Puffer (pH 7,5) gefroren aufbewahrt. Die Bestimmung der spezifischen Aktivität erfolgt nach Bestimmung der Proteinkonzentration entsprechend der Vorschrift von Folk,J.E. (Meth. Enzym., 19:504-508, 1970). Geht man von Material aus, daß gemäß Beispiel 3 hergestellt wurde, so werden nur 15 mg des Ausgangsmaterial zur Aktivierung verwendet.

### Beispiel 5

Das Beispiel beschreibt den kombinierten Einsatz von Trypsin und Procarboxypeptidase B zur Herstellung von Insulin aus Mono- Arg - Insulin. Beispiel 6 der Europäischen Patentanmeldung EP-A 0 347 781 beschreibt die Umsetzung von Mono - Arg - Insulin mit Trypsin und Carboxypeptidase B in ein und demselben Reaktionsgefäß. Im vorliegenden Beispiel wird nun die Carboxypeptidase B in Beispiel 6 der Europäischen Patentanmeldung EP-A 0 347 781 durch 15 µg Procarboxypeptidase B aus Beispiel 2 dieser Anmeldung bzw. durch 10 µg Procarboxypeptidase B aus Beispiel 3 dieser Anmeldung ersetzt. In beiden Reaktionen wird die Trypsinkonzentration durch Einsatz von 3µl Trypsin anstelle von 2,5 µl der Stammlösung (gemäß Beispiel 6 der Europäischen Patentanmeldung EP-A 0 347 781) erhöht.

## Patentansprüche

1. Verfahren zur Herstellung von reifem Insulin oder einem reifen Insulinderivat, wobei
(a) eine natürliche oder unnatürliche Vorläuferform von Carboxypeptidase B oder einer Isoform oder eines Muteins der Carboxypeptidase B in einem Mikroorganismus sekretorisch exprimiert,
(b) die sekretorisch exprimierte Vorläuferform gereinigt und
(c) die gereinigte Vorläuferform durch eine enzymatische Behandlung in die aktive Carboxypeptidase B oder die Isoform oder das Mutein von Carboxypeptidase B überführt wird,
wobei Schritt (c) unter geeigneten Reaktionsbedingungen in Gegenwart einer Insulinvorläuferform enthaltend die B, C und A-Ketten von Insulin oder eines Insulinderivats abläuft und hierbei reifes Insulin oder ein reifes Insulinderivat entsteht, welches aus dem Reaktionsgemisch isoliert wird.

2. Verfahren gemäß Anspruch 1, wobei die pankreatische Carboxypeptidase B oder eine Isoform davon aus dem Menschen stammt.

3. Verfahren gemäß Anspruch 1 oder 2, wobei die natürliche Vorläuferform der Procarboxypeptidase B oder einer Isoform der Procarboxypeptidase B entspricht.

4. Verfahren gemäß Anspruch 1 oder 2, wobei die unnatürliche Vorläuferform von Carboxypeptidase B folgende Struktur hat:
S-(As)ₓ-E-CB, (I)
wobei
S ein Signalpeptid ist, welches die Sekretion des bei der Expression entstehenden Fusionsproteins aus dem jeweiligen Mikroorganismus bewirkt;
As eine beliebige genetisch kodierbare Aminosäure ist;
E ein peptidischer Linker bestehend aus einer Endopeptidaseerkennungssequenz ist;
CB die Aminosäuresequenz der Carboxypeptidase B oder einer Isoform oder eines Muteins der Carboxypeptidase B ist; und
x eine ganze Zahl von 0 - 100 ist.

5. Verfahren gemäß einem der Ansprüche 1 bis 4, wobei an die natürliche oder unnatürliche Vorläuferform eine Peptidsequenz angefügt ist, welche die affinitätschromatographische Reinigung der Vorläuferform ermöglicht.

6. Verfahren gemäß Anspruch 5, wobei die für die affinitätschromatographische Reinigung angefügte Sequenz 1 bis 6, vorzugsweise 4 Histidinreste darstellt.

7. Verfahren gemäß einem der Ansprüche 1 bis 6, wobei als Mikroorganismus zur Expression die Hefe *Pichia pastoris* verwendet wird.

8. Verfahren gemäß einem der Ansprüche 1 bis 7, wobei zur enzymatischen Behandlung die Enzyme Trypsin, Elastase, Faktor Xa, Chymotrypsin oder Collagenase, vorzugsweise Trypsin, verwendet werden.

## Claims

1. A process for the preparation of mature insulin or a mature insulin derivative, where
(a) a natural or unnatural precursor form of carboxypeptidase B or an isoform or a mutein of carboxypeptidase B is expressed in a microorganism by secretion,
(b) the precursor form expressed by secretion is purified and
(c) the purified precursor form is converted into the active carboxypeptidase B or the isoform or the mutein of carboxypeptidase B by means of an enzymatic treatment,
where step (c) proceeds under suitable reaction conditions in the presence of an insulin precursor form comprising the B, C and A chains of insulin or of an insulin derivative and in this process mature insulin or a mature insulin derivative is formed, which is isolated from the reaction mixture.

2. The process as claimed in claim 1, wherein the pancreatic carboxypeptidase B or an isoform thereof originates from man.

3. The process as claimed in claim 1 or 2, where the natural precursor form corresponds to procarboxypeptidase B or an isoform of procarboxypeptidase B.

4. The process as claimed in claim 1 or 2, where the unnatural precursor form of carboxypeptidase B has the following structure:
S-(As)ₓ-E-CB, (I)
where
S is a signal peptide which brings about the secretion, from the respective microorganism, of the fusion protein formed during expression;
As is any desired genetically encodable amino acid;
E is a peptide linker consisting of an endopeptidase recognition sequence;
CB is the amino acid sequence of carboxypeptidase B or an isoform or a mutein of carboxypeptidase B; and
x is an integer from 0-100.

5. The process as claimed in one of claims 1 to 4, where a peptide sequence is attached to the natural or unnatural precursor form which makes possible the purification of the precursor form by affinity chromatography.

6. The process as claimed in claim 5, where the sequence attached for purification by affinity chromatography is 1 to 6, preferably 4, histidine residues.

7. The process as claimed in one of claims 1 to 6, where the microorganism used for expression is the yeast *Pichia pastoris.*

8. The process as claimed in one of claims 1 to 7, where the enzymes trypsin, elastase, factor Xa, chymotrypsin or collagenase, preferably trypsin, are used for the enzymatic treatment.

## Revendications

1. Procédé de préparation d'insuline mûre ou d'un dérivé mûr d'insuline, dans lequel
(a) une forme précurseur naturelle ou non naturelle de carboxypeptidase B ou de son isoforme ou d'une mutéine de la carboxypeptidase B est exprimée sécrétoirement dans un microorganisme;
(b) la forme précurseur sécrétoirement exprimée est purifiée, et
(c) la forme précurseur purifiée est transformée par traitement enzymatique en la carboxypeptidase B active ou l'isoforme ou la mutéine de la carboxypeptidase B,
l'étape (c) se déroulant dans des conditions réactionnelles appropriées en présence d'une forme précurseur d'insuline contenant les chaînes B, C et A de l'insuline ou d'un dérivé d'insuline et à cette occasion, l'insuline mûre ou un dérivé mûr d'insuline est produit, qui est isolé du mélange réactionnel.

2. Procédé selon la revendication 1, dans lequel la carboxypeptidase B pancréatique ou une isoforme de celle-ci provient de l'homme.

3. Procédé selon la revendication 1 ou 2, dans lequel la forme précurseur naturelle correspond à la procarboxypeptidase B ou à une isoforme de la procarboxypeptidase B.

4. Procédé selon la revendication 1 ou 2, dans lequel la forme précurseur non naturelle de la carboxypeptidase B présente la structure suivante :
S-(As)ₓ-E-CB (I)
dans laquelle :
S est un peptide de signal, lequel active la sécrétion de la protéine de fusion, du microorganisme respectif, apparue lors de l'expression;
As est n'importe quel acide aminé pouvant être codé génétiquement;
E est un lieur peptidique composé d'une séquence de reconnaissance de l'endopeptidase;
CB est la séquence d'acides aminés de la carboxypeptidase B ou de son isoforme ou d'une mutéine de la carboxypeptidase B, et
x est un nombre entier de 0 à 100.

5. Procédé selon l'une des revendications 1 à 4, dans lequel une séquence de peptides est ajoutée à la forme précurseur naturelle ou non naturelle, laquelle permet la purification par chromatographie d'affinité.

6. Procédé selon la revendication 5, dans lequel la séquence ajoutée pour la purification par chromatographie d'affinité représente 1 à 6, de préférence 4 radicaux d'histidine.

7. Procédé selon l'une des revendications 1 à 6, dans lequel on utilise comme microorganisme d'expression la levure *Pichia pastoris.*

8. Procédé selon l'une des revendications 1 à 7, dans lequel on utilise pour le traitement enzymatique les enzymes trypsine, élastase, facteur Xa, chymotrypsine ou collagénase, de préférence la trypsine.
